# EUROPÄISCHE PATENTSCHRIFT

(11) **EP 0 831 080 B1**
(45) Veröffentlichungstag und Bekanntmachung des Hinweises auf die Patenterteilung: **05.09.2001**
(21) Anmeldenummer: 97115511.4
(22) Anmeldetag: 08.09.1997
(51) Int. Cl.: C07C 29/20, C07C 31/137

(54) **Verfahren zur Herstellung von Isocamphyl-cyclohexanolen**
Method for the preparation of isocamphyl-cyclohexanols
Procédé pour la préparation d'isocamphyl-cyclohexanols

(30) Priorität: 19.09.1996 DE 19638300
(43) Veröffentlichungstag der Anmeldung: 25.03.1998
(73) Patentinhaber: BAYER AG, 51368 Leverkusen (DE)
(72) Erfinder: Darsow, Gerhard, Dr., 47804 Krefeld (DE)

(56) Entgegenhaltungen:
- DD-A- 224 315
- DE-A- 2 921 139
- US-A- 4 014 944
- CHEMICAL ABSTRACTS, vol. 51, no. 21, 10. November 1957 Columbus, Ohio, US; abstract no. 17107c, L.A. KHELFITS, ET AL.: "Industrial method for the manufacture of santalidol" XP002038808 & MASOLBOINO-ZHIROVAYA PROM., Bd. 23, Nr. 6, 1957, Seiten 35-38,

## Beschreibung

Die Erfindung betrifft ein neues, kontinuierliches Verfahren zur Herstellung von Isocamphyl-cyclohexanolen aus Verbindungen, die das Kohlenstoffgerüst der Isocamphyl-guajakole bzw. Isocamphyl-phenole besitzen, durch Hydrierung mit Wasserstoff unter Einsatz von Katalysatoren.

Isocamphyl-cyclohexanole sind Bestandteile der industriell erzeugten Sandelholzduftstoffe, die aus synthetischen Mischungen verschiedener Terpencyclohexanol-Isomeren bestehen. Industrielle Sandelholzduftstoffe ersetzen natürliches Sandelholzöl in Seifen, Kosmetikartikeln und Parfümkompositionen.

Als Ausgangsverbindungen für die Isocamphyl-cyclohexanole können z. B. die Isocamphyl-guajakole in bekannter Weise durch Reaktion von Camphen mit Guajakol unter Mithilfe von sauren Katalysatoren, z. B. Bortrifluorid und Essigsäure, hergestellt werden; die Isocamphyl-guajakole werden dann durch Hydrierung des Aromatkerns und Abspaltung der Methoxygruppe in die Isocamphyl-cyclohexanole umgewandelt.

Die Reaktionen verlaufen nach folgender Gleichung:

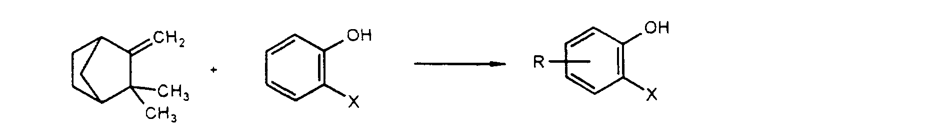

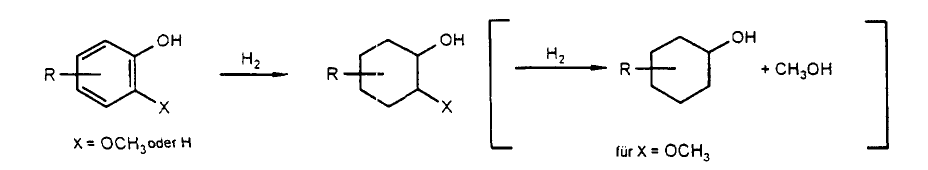

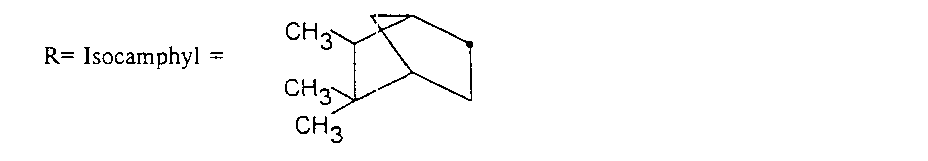

Es ist bereits bekannt, Alkylierungsgemische aus Camphen und Catechol (US 4 014 944) bzw. Camphen und Guajakol (DE-A 2 921 139) mit Wasserstoff über Raney-Nickel zu den gewünschten Isocamphyl-cyclohexanolen zu hydrieren.

Diese Verfahren des Standes der Technik arbeiten ausnahmslos diskontinuierlich mit pulverförmigen Katalysatoren nach dem Suspensionsverfahren.

Bei Pulverkatalysatoren bestehen die folgenden Schwierigkeiten: (1) diese Katalysatoren gezielt und gleichmäßig zu aktivieren, (2) Pulverkatalysatoren mit Hilfe von speziellen Schlammpumpen umzuwälzen und (3) Pulverkatalysatoren vom Reaktionsprodukt quantitativ abzutrennen. Schlammpumpen unterliegen nämlich einer hohen mechanischen Beanspruchung. Die quantitative Entfernung pulverförmiger Katalysatoren ist weiterhin aufwendig, weil sie eine Grob- und eine Feinfiltration mit Apparaten in Wechselausführung erfordert. Ferner ist die Gefahr groß, daß die Katalysatoren durch diese zusätzlichen Operationen schnell ihre Aktivität verlieren und daher weiterhin hohe Katalysatorverbräuche verursachen. Ferner sind Pulverkatalysatoren nur begrenzt belastbar und nur schwierig von den Reaktionsprodukten zu befreien, was ihre Aufarbeitung erschwert.

Es bestand daher die Aufgabe, hoch belastbare, säurefeste und langlebige Katalysatoren für die Herstellung von Isocamphyl-cyclohexanolen herzustellen, die frei von komplizierten Trägersystemen und daher wieder aufarbeitbar sein sollten.

Überraschenderweise läßt sich das geschilderte Problem mit Hilfe trägerfreier Festbettkatalysatoren lösen, die als verpreßte aus Metallpulvern oder legierten Metallpulvern hergestellte Formkörper vorliegen, die eine Druckfestigkeit von 20 bis 250 N und eine innere Oberfläche von 10 bis 90 m²/g aufweisen und bei denen die Metallpulver 60 bis 100 Gew.-% eines oder mehrerer Eisenmetalle, 0 bis 15 Gew.-% eines oder mehrerer Metalle der VI. Nebengruppe und 0 bis 25 Gew.-% eines oder mehrerer hydrierinerter Elemente aus der Gruppe von Aluminium, Silicium, Titan und Kohlenstoff, alles bezogen auf das Gesamtgewicht des Metallpulvers, enthalten.

Gegenstand der Erfindung ist damit ein kontinuierliches Verfahren zur Herstellung von Isocamphyl-cyclohexanolen aus Verbindungen, die das Kohlenstoffgerüst der Isocamphyl-guajakole oder Isocamphyl-phenole besitzen, durch Hydrierung mit Wasserstoff bei erhöhter Temperatur und erhöhtem Druck, dadurch gekennzeichnet, daß als Katalysatoren trägerfreie Formkörper verwendet werden, die als verpreßte, aus Metallpulvern hergestellte Formkörper vorliegen, die eine Druckfestigkeit von 20 - 250 N und eine innere Oberfläche von 10 - 90 m²/g aufweisen und bei denen die Metallpulver 60 - 100 Gew.-% eines oder mehrerer Eisenmetalle, 0 - 15 Gew.-% eines oder mehrerer Metalle der VI. Nebengruppe und 0 - 25 Gew.-% eines oder mehrerer hydrierinerter Elemente aus der Gruppe von Aluminium, Silicium, Titan und Kohlenstoff, alles bezogen auf das Gesamtgewicht des Metallpulvers, enthalten.

Unter Verbindungen, die das Kohlenstoffgerüst der Isocamphyl-guajakole oder Isocamphyl-phenole besitzen, sind gegebenenfalls substituierte Isocamphyl-guajakole und -phenole zu verstehen, wobei als Substituenten vorzugsweise Alkylgruppen, insbesondere C₁-C₆-Alkylgruppen in Frage kommen.

Die erfindungsgemäß einzusetzenden Metallpulver können die genannten Elemente der Eisengruppe sowie gegebenenfalls der VI. Nebengruppe und gegebenenfalls die hydriernierten Elemente Al, Si, Ti und C in elementarer Form oder in Form von Legierungen enthalten.

Die Eisenuntergruppe der VIII. Nebengruppe des Periodensystems (Mendelejew) enthält die Elemente Eisen, Kobalt und Nickel. Die erfindungsgemäß zu verwendenden trägerfreien Formkörper enthalten eines oder mehrere dieser Metalle in Mengen von mindestens 60, vorzugsweise mindestens 70, insbesondere mindestens 80 Gew.-%, bezogen auf das Gesamtgewicht der trägerfreien Formkörper.

Die VI. Nebengruppe des Periodensystems enthält die Elemente Chrom, Molybdän und Wolfram. Die erfindungsgemäß zu verwendenden trägerfreien Formkörper enthalten eines oder mehrere dieser Metalle in Mengen von 0 - 15 Gew.-%. Für den Fall ihres Vorliegens enthalten die Metallpulver oder legierten Metallpulver mindestens 0,1, vorzugsweise mindestens 0,3, insbesondere vorzugsweise mindestens 0,5 Gew.-%, bezogen auf die trägerfreien Formkörper; sie enthalten eines oder mehrere dieser Metalle in Mengen von höchstens 15, vorzugsweise höchstens 10 und insbesondere höchstens 5 Gew.-%, bezogen auf die trägerfreien Formkörper.

Die erfindungsgemäß zu verwendenden trägerfreien Formkörper enthalten darüber hinaus - jeweils bezogen auf die trägerfreien Formkörper -0 bis 25, vorzugsweise 0 bis 15 Gew.-% eines oder mehrerer Elemente aus der Gruppe Aluminium, Silicium, Titan und Kohlenstoff. Diese Elemente sind unter den Bedingungen des erfindungsgemäßen Verfahrens hydrierinert, d.h. sie wirken nicht katalytisch. Nach einer bevorzugten Ausführungsform enthalten die trägerfreien Formkörper außer den Metallen der VIII. und gegebenenfalls VI. Nebengruppe nicht mehr als 10 Gew.-% Aluminium und nicht mehr als 5 Gew.-% je Element Silicium, Titan und Kohlenstoff.

Die Herstellung der trägerfreien Formkörper kann nach gebräuchlichen Methoden durch Verpressen der Metallpulver oder legierten Metallpulver auf Tablettier- und Pelletiermaschinen unter hohem Druck erfolgen, wobei zur Verbesserung des Haftvermögens der Metallpartikel auch Graphit in Mengen von 0,5 - 1,5 Gew.-% bezogen auf das Gesamtgewicht der den Katalysator bildenden Bestandteile, oder Klebestoffe in kleinen Mengen zum Einsatz kommen können. Die Herstellung der trägerfreien Formkörper erfolgt vorzugsweise in einer sauerstofffreien Atmosphäre, um Oberflächenoxidationen zu vermeiden. Am wirksamsten und für die Reaktionsführung am günstigsten sind tablettierte und pelletierte Formkörper mit Durchmessern von 3 bis 7 mm. Von erheblicher Bedeutung ist die Druckfestigkeit der Formkörper, die erfindungsgemäß bei Werten von 20 bis 250 N, bevorzugt 100 bis 220 N, liegt. Niedrigere Druckfestigkeiten führen zu Formkörperzerfall bzw. erosivem Abrieb, was eine metallische Kontaminierung des Reaktionsproduktes bewirken würde. Höhere Werte bedingen einen unangemessenen Aufwand beim Verpressen, ohne daß weitere Vorteile erzielt werden. Von erheblicher Bedeutung ist weiterhin die innere Oberfläche der Formkörper, die erfindungsgemäß bei Werten von 10 bis 90 m²/g liegt und ausschlaggebend für einen möglichst quantitativen Umsatz der Einsatzstoffe ist.

Die Druckfestigkeit der trägerfreien Formkörper kann nach DIN 50 106 bestimmt werden.

Die Überprüfung von trägerfreien Formkörpern auf die anspruchsgemäßen inneren Oberflächen und damit auf Brauchbarkeit für das erfindungsgemäße Verfahren können nach Methoden durchgeführt werden, die von F M. Nelsen und F. T. Eggertsen, Analyt. Chem. 30 (1958), S. 1387-1390 bzw. S.J. Gregg und K.S.W. Sing, Adsorption, Surface Area and Porosity, London 1982, Kap. 2 und 6, beschrieben worden sind.

Der Hydrierreaktor kann entweder ein einzelnes Hochdruckrohr aus Stahl oder einer Stahllegierung sein, das mit den trägerfreien Formkörpern ganz oder teilweise gefüllt wird, wobei bei gewissen Rohrquerschnitten auch die Anwendung der trägerfreien Formkörper auf Horden (Drahtkörbe oder ähnliches) nützlich sein kann, oder aber ein ummanteltes Hochdruckrohrbündel, dessen Einzelrohre mit trägerfreien Formkörpern ganz oder teilweise gefüllt werden. Weiterhin kann anstelle eines größeren Einzelrohrreaktors eine Anordnung von mehreren kleinen Einzelreaktoren hintereinander in einer Kaskade betrieben werden.

Für den Hydrierprozeß wird auf einen Druck von 50 - 400 bar, bevorzugt 100 bis 350 bar, besonders bevorzugt 150 - 300 bar, vorkomprimierter reiner Wasserstoff eingesetzt, wobei man mit einer 10- bis 60-fachen, bevorzugt 20- bis 40-fachen molaren Wasserstoffmenge, bezogen auf die stöchiometrische Menge, arbeitet.

Die Hydrierung erfolgt kontinuierlich im Festbettverfahren an den als Hydrierkatalysatoren dienenden trägerfreien Formkörpern in der absteigenden Flüssigphase oder aber bevorzugt in der aufsteigenden Flüssigphase, indem man die zu hydrierende Lösung entweder im Gleichstrom von unten aufsteigend gemeinsam mit dem vorher zugemischten Wasserstoff über den in einem Hydrierreaktor angebrachten Katalysator strömen läßt (Gleichstromverfahren) oder aber, indem man die von unten aufsteigende zu hydrierende Lösung dem von oben einströmenden Wasserstoff entgegenführt (Gegenstromverfahren).

Der Temperaturbereich für das erfindungsgemäße Verfahren beträgt 140 bis 280°C, bevorzugt 180 bis 260°C.

Mit Hilfe des Einsatzes der beschriebenen Katalysatoren entsteht im erfindungsgemäßen Verfahren aus den eingesetzten Isocamphyl-guajakolen bzw. Isocamphylphenolen ein Gemisch isomerer Isocamphyl-cyclohexanole, wie 2-Hydroxy-1-(5-isocamphyl)-cyclohexan, 3-Hydroxy-1-(5-isocamphyl)-cyclohexan, 4-Hydroxy-1-(5-isocamphyl)-cyclohexan.

Bei der beschriebenen Verfahrensweise beträgt die Katalysatorbelastung 0,05 bis 1,0 kg, bevorzugt 0,1 bis 0,5 kg Einsatzprodukt pro Liter Katalysator und Stunde. Die eingesetzten Isocamphyl-guajakole bzw. Isocamphyl-phenole können mit einem geeigneten reaktionsinerten Lösungsmittel, beispielsweise aliphatischen Monoalkoholen oder Cyclohexanol in einer Menge von 10 bis 100, bevorzugt 10 bis 40 Gew.-%, bezogen auf das Gewicht des Einsatzproduktes, verdünnt werden.

Das den Hydrierreaktor verlassende Reaktionsgemisch wird nach Abkühlung auf eine Temperatur <60°C entspannt, wobei man den überschüssigen Wasserstoff abfangen und nach erfolgtem Komprimieren und Ergänzung von verbrauchtem Wasserstoff erneut zum Einsatz bringen kann.

Unter den geschilderten Reaktionsbedingungen sind auf diese Weise ganz unerwartet hohe Katalysatorstandzeiten von 15.000 Stunden und mehr zu erzielen, was zu Katalysatorverbräuchen <0,1 Gew.-%, bezogen auf das hergestellte Reaktionsprodukt, führt.

Die erfindungsgemäß einzusetzenden sauerstofffreien und trägerfreien Festbettkatalysatoren neigen im Gegensatz zu trägerhaltigen Katalysatoren nicht zum "Ausbluten", d. h. nicht zum Übergang von Katalysatorbestandteilen in ionischer oder kolloidaler Form in die Lösungsphase des Substrats, so daß das Substrat nicht durch Schwermetalle kontaminiert wird, die normalerweise ebenfalls nur mühsam, beispielsweise mit Hilfe von Ionenaustauschern, aus dem Substrat entfernt werden können. Die einzusetzenden Katalysatormetalle können, etwa nach längerem Gebrauch des Katalysators, leicht aufgearbeitet und wiederverwendet werden, da die Schwermetalle nicht umständlich von einem Trägermaterial getrennt werden müssen.

Die erhaltenen Reaktionsprodukte sind praktisch frei von aromatischen Anteilen.

Nach der Hydrierung ohne Lösungsmittel können die erhaltenen Isocamphyl-cyclohexanole nach destillativer Entfernung eventuell gebildeter Leichtsieder normalerweise ohne weiteren Reinigungsprozeß, bei der Hydrierung mit Lösungsmittel nach zusätzlicher Abdestillation des Lösungsmittels, weiterverarbeitet werden. Es ist jedoch auch möglich, die erhaltenen Isocamphyl-cyclohexanole destillativ oder mit anderen bekannten physikalischen Trennmethoden zu trennen und aufzukonzentrieren.

### Beispiele

### Beipiel 1

Ein senkrecht stehendes, wärmeisoliertes Hochdruckrohr aus nichtrostendem, säurefestem Stahl von 45 mm Innendurchmesser und 1 m Länge, das vorher mit Stickstoff sauerstofffrei gespült worden war, wurde mit 1,4 l eines durch Tablettierung einer pulverisierten Ni/Mo/Al-Legierung mit einem Mo-Gehalt von 1,02 Gew.-% und einem Al-Gehalt von 6,1 Gew.-% hergestellten Katalysators gefüllt.

Die Tabletten hatten bei einer Zylinderhöhe von 5 mm und einem Durchmesser von 5 mm eine Druckfestigkeit von 210 N auf die Zylindermantelfläche und eine innere Oberfläche von 71 m²/g.

Anschließend wurden stündlich 180 g einer 38 Gew.-%igen Lösung eines Gemisches aus Isocamphyl-guajakolen, wie es bei der Alkylierung von Camphen mit Guajakol anfällt, in Cyclohexanol gemeinsam mit 1,5 Nm³ Wasserstoff unter einem Druck von 300 bar von unten nach oben aufsteigend durch das Hochdruckrohr gepumpt, wobei die zu hydrierende Mischung vor Eintritt in das Hochdruckrohr in einem vorgeschalteten elektrisch beheizten Wärmeaustauscher auf eine Temperatur von 190°C erhitzt wurde.

Das das Reaktionsrohr verlassende Reaktionsprodukt wurde in einem zweiten Wärmeaustauscher (Wasserkühler) unter 300 bar Wasserstoffdruck auf eine Temperatur <60°C abgekühlt und in einem Gasabscheider vom überschüssigen Wasserstoff, der wieder in das Reaktionssystem zurückgeführt wurde, getrennt.

Nach weiterer Abkühlung auf eine Temperatur <30°C und Entspannung auf Normaldruck wurde das Reaktionsprodukt UV-spektroskopisch untersucht.

Es wurde festgestellt, daß der Restaromatanteil bei weniger als 0,1 Gew.-% lag.

Nach dem Abdestillieren des Lösungsmittels wurde ein glasklares viskoses Öl erhalten, das die isomeren Isocamphylverbindungen
2-Hydroxy-1-(5-isocamphyl)-cyclohexan,
3-Hydroxy-1-(5-isocamphyl)-cyclohexan und
4-Hydroxy-1-(5-isocamphyl)-cyclohexan
im Mischungsverhältnis von ca. 3:1:3 enthielt und angenehm nach Sandelholz duftete.

Der Katalysator war nach einer Laufzeit von 5.018 Stunden unverändert wirksam.

### Beispiel 2

Ein senkrecht stehendes, wärmeisoliertes Hochdruckrohr aus nichtrostendem, säurefestem Stahl von 45 mm Innendurchmesser und 1 m Länge das vorher mit Stickstoff sauerstofffrei gespült worden war, wurde mit 1,4 l eines durch Tablettierung einer pulverisierten Ni/Al-Legierung mit einem Al-Gehalt von 5,8 Gew.-% hergestellten Katalysators gefüllt.

Die Tabletten hatten bei einer Zylinderhöhe von 5 mm und einem Durchmesser von 5 mm eine Druckfestigkeit von 156 N auf die Zylindermantelfläche und eine innere Oberfläche von 69 m²/g.

Durch dieses Rohr wurden 170 g einer 38 Gew.-%igen Lösung eines Gemisches aus Isocamphyl-guajakolen, wie es bei der Alkylierung von Camphen mit Guajakol anfällt, in Cyclohexanol gemeinsam mit 1,5 Nm³ Wasserstoff unter einem Druck von 300 bar von unten nach oben aufsteigend durch das Hochdruckrohr gepumpt, wobei die zu hydrierende Mischung vor Eintritt in das Hochdruckrohr in einem vorgeschalteten elektrisch beheizten Wärmeaustauscher auf eine Temperatur von 195°C erhitzt wurde.

Das das Reaktionsrohr verlassende Reaktionsprodukt wurde in einem zweiten Wärmeaustauscher (Wasserkühler) unter 300 bar Wasserstoffdruck auf eine Temperatur <60°C abgekühlt und in einem Gasabscheider vom überschüssigen Wasserstoff, der wieder in das Reaktionssystem zurückgeführt wurde, getrennt.

Nach weiterer Abkühlung auf eine Temperatur <30°C und Entspannung auf Normaldruck wurde das Reaktionsprodukt UV-spektroskopisch untersucht.

Es wurde festgestellt, daß der Restaromatanteil bei weniger als 0,1 Gew.-% lag.

Nach dem Abdestillieren des Lösungsmittels wurde ein glasklares viskoses Öl erhalten, das die isomeren Isocamphylverbindungen
2-Hydroxy-1-(5-isocamphyl)-cyclohexan,
3-Hydroxy-1-(5-isocamphyl)-cyclohexan und
4-Hydroxy-1-(5-isocamphyl)-cyclohexan,
im Mischungsverhältnis von ca. 3:1:2 enthielt und angenehm nach Sandelholz duftete.

Der Katalysator war nach einer Laufzeit von 8.608 Stunden unverändert wirksam.

### Beispiel 3

Ein senkrechtes stehendes, wärmeisoliertes Hochdruckrohr aus nichtrostendem Stahl von 45 mm Innendurchmesser und 1 m Länge wurde mit 1,4 l eines durch Tablettierung einer pulverisierten Ni/Fe-Legierung gewonnenen Katalysators gefüllt.

Die Legierung enthielt einen Fe-Anteil von 15 %. Die Tabletten hatten bei einer Zylinderhöhe von 5 mm und einem Durchmesser von 5 mm eine Druckfestigkeit von 137 N auf die Zylindermantelfläche und eine innere Oberfläche von 74 m²/g.

Anschließend wurden stündlich 180 g einer 38 gew.-%igen Lösung eines Gemisches aus Isocamphyl-guajakolen, wie es bei der Alkylierung von Camphen mit Guajakol anfällt, in Cyclohexanol gemeinsam mit 1,5 Nm³ Wasserstoff unter einem Druck von 300 bar von unten nach oben aufsteigend durch das Hochdruckrohr gepumpt, wobei die zu hydrierende Mischung vor Eintritt in das Hochdruckrohr in einem vorgeschalteten elektrisch beheizten Wärmeaustauscher auf eine Temperatur von 205°C erhitzt wurde.

Das das Reaktionsrohr verlassende Reaktionsprodukt wurde in einem zweiten Wärmeaustauscher (Wasserkühler) unter 300 bar Wasserstoffdruck auf eine Temperatur <60°C abgekühlt und in einem Gasabscheider vom überschüssigen Wasserstoff, der wieder in das Reaktionssystem zurückgeführt wurde, getrennt.

Nach weiterer Abkühlung auf eine Temperatur <30°C und Entspannung auf Normaldruck wurde das Reaktionsprodukt UV-spektroskopisch untersucht.

Es wurde festgestellt, daß der Restaromatanteil bei weniger als 0,1 Gew.-% lag.

Nach dem Abdestillieren des Lösungsmittels wurde ein glasklares viskoses Öl erhalten, das die isomeren Isocamphyl-verbindungen
2-Hydroxy-1-(5-isocamphyl)-cyclohexan,
3-Hydroxy-1-(5-isocamphyl)-cyclohexan und
4-Hydroxy-1-(5-isocamphyl)-cyclohexan,
im Mischungsverhältnis von ca. 3:2:3 enthielt und angenehm nach Sandelholz duftete.

Der Katalysator war nach einer Laufzeit von 4.100 Stunden unverändert wirksam, so daß sich die Zusammensetzung des Reaktionsproduktes über diesen Zeitraum nicht veränderte.

## Patentansprüche

1. Kontinuierliches Verfahren zur Herstellung von Isocamphyl-cyclohexanolen aus den Verbindungen Isocamphyl-guajakol oder Isocamphyl-phenol durch Hydrierung mit Wasserstoff bei erhöhter Temperatur und erhöhtem Druck, **dadurch gekennzeichnet**, daß als Katalysatoren dienende trägerfreie Materialien eingesetzt werden, die als verpreßte aus Metallpulvern hergestellte Formkörper vorliegen, die eine Druckfestigkeit von 20 bis 250 N und eine innere Oberfläche von 10 bis 90 m²/g aufweisen und bei denen die Metallpulver 60 bis 100 Gew.-% eines oder mehrerer Eisenmetalle, 0 bis 15 Gew.-% eines oder mehrerer Metalle der VI. Nebengruppe und 0 bis 25 Gew.-% eines oder mehrerer hydrierinerter Elemente aus der Gruppe von Aluminium, Silicium, Titan und Kohlenstoff, alles bezogen auf das Gesamtgewicht des Metallpulvers, enthalten.

2. Verfahren nach Anspruch 1, **dadurch gekennzeichnet**, daß die Metallpulver 70 bis 100 Gew.-%, bevorzugt 80 bis 100 Gew.-%, eines oder mehrerer Eisenmetalle enthalten.

3. Verfahren nach Anspruch 1, **dadurch gekennzeichnet**, daß die Metallpulver einen Gehalt von 0,1 bis 15 Gew.-%, bevorzugt von 0,3 bis 10 Gew.-%, besonders bevorzugt von 0,5 bis 5 Gew.-% eines oder mehrerer Metalle der VI. Nebengruppe enthalten.

4. Verfahren nach Anspruch 1, **dadurch gekennzeichnet**, daß die Metallpulver einen Gehalt von 0 bis 10 Gew.-% an Aluminium und von 0 bis 5 Gew.-% je Element Si, Ti und C enthalten.

5. Verfahren nach Anspruch 4, **dadurch gekennzeichnet**, daß der Gesamtgehalt, der Elemente Al, Si, Ti und C 0 bis 15 Gew.-%, bevorzugt 0 bis 10 Gew.-%, beträgt.

6. Verfahren nach Anspruch 1, **dadurch gekennzeichnet**, daß der Wasserstoffdruck 5 bis 400 bar, bevorzugt 100 bis 350 bar, besonders bevorzugt 100 bis 300 bar, beträgt.

7. Verfahren nach Anspruch 1, **dadurch gekennzeichnet**, daß die Hydriertemperatur 140 bis 280°C, bevorzugt 180 bis 260°C beträgt.

8. Verfahren nach Anspruch 1, **dadurch gekennzeichnet**, daß während des Verfahrens pro Mol Ausgangsmaterial eine 10- bis 60-fache, bevorzugt 20- bis 40-fache höhere Wasserstoffmenge, bezogen auf die stöchimetrische Menge, eingesetzt wird.

9. Verfahren nach Anspruch 1, **dadurch gekennzeichnet**, daß kontinuierlich in der herabsteigenden Flüssigphase oder bevorzugt in der aufsteigenden Flüssigphase an fest angeordneten Katalysatoren gearbeitet wird und eine Katalysatorbelastung von 0,05 bis 1,0 kg, bevorzugt von 0,1 bis 0,5 kg Ausgangsprodukt, pro Liter Katalysator und Stunde eingestellt wird.

10. Verfahren nach Anspruch 1, **dadurch gekennzeichnet**, daß das Ausgangsprodukt mit 10 bis 100 Gew.-%, bevorzugt 10 bis 40 Gew.-% eines reaktionsinerten Lösungsmittels, bezogen auf das Ausgangsprodukt, verdünnt wird.

## Claims

1. Continuous method for the preparation of isocamphyl cyclohexanols from the compounds isocamphyl guaiacol or isocamphyl phenol by hydrogenation with hydrogen at elevated temperature and elevated pressure, **characterised in that** support-free materials serving as catalysts are used which are in the form of compressed shaped bodies produced from metal powders, which exhibit a compressive strength of 20 to 250 N and an internal surface area of 10 to 90 m²/g, and in which the metal powders contain 60 to 100 wt.% of one or more ferrous metals, 0 to 15 wt.% of one or more metals belonging to the 6^{th} subgroup and 0 to 25 wt.% of one or more hydrogenation-inert elements from the group comprising aluminium, silicon, titanium and carbon, relative in each case to the total weight of metal powder.

2. Method according to claim 1, **characterised in that** the metal powders contain 70 to 100 wt.%, preferably 80 to 100 wt.%, of one or more ferrous metals.

3. Method according to claim 1, **characterised in that** the metal powders contain a content of 0.1 to 15 wt.%, preferably 0.3 to 10 wt.%, particularly preferably 0.5 to 5 wt.% of one or more metals belonging to the 6^{th} subgroup.

4. Method according to claim 1, **characterised in that** the metal powders contain a content of 0 to 10 wt.% of aluminium and 0 to 5 wt.% of each element Si, Ti and C.

5. Method according to claim 4, **characterised in that** the total content of elements Al, Si, Ti and C is 0 to 15 wt.%, preferably 0 to 10 wt.%.

6. Method according to claim 1, **characterised in that** the hydrogen pressure is 5 to 400 bar, preferably 100 to 350 bar, particularly preferably 100 to 300 bar.

7. Method according to claim 1, **characterised in that** the hydrogenation temperature is 140 to 280°C, preferably 180 to 260°C.

8. Method according to claim 1, **characterised in that** a 10 to 60 times, preferably 20 to 40 times greater quantity of hydrogen relative to the stoichiometric quantity is used during the process per mol of starting material.

9. Method according to claim 1, **characterised in that** the method is performed continuously in the descending liquid phase or preferably in the ascending liquid phase on fixed catalysts and a catalyst load of 0.05 to 1.0 kg, preferably 0.1 to 0.5 kg of starting product is established per litre of catalyst and per hour.

10. Method according to claim 1, **characterised in that** the starting product is diluted with 10 to 100 wt.%, preferably 10 to 40 wt.% of a reaction-inert solvent, relative to the starting product.

## Revendications

1. Procédé en continu pour la préparation d'isocamphyl-cyclohexanols à partir des composés d'isocamphyl-gayacol ou d'isocamphyl-phénol par hydrogénation avec de l'hydrogène à température élevée et sous pression élevée, **caractérisé en ce qu**'on met en oeuvre des matières sans support faisant office de catalyseurs qui sont présentes sous la forme de corps moulés comprimés préparés à partir de poudres métalliques, qui présentent une résistance à la pression de 20 à 250 N et une surface interne de 10 à 90 m²/g et dans lesquels les poudres métalliques contiennent un ou plusieurs métaux du fer à concurrence de 60 à 100 % en poids, un ou plusieurs métaux du VIème sous-groupe à concurrence de 0 à 15 % en poids et un ou plusieurs éléments inertes vis-à-vis de l'hydrogénation choisis parmi le groupe comprenant l'aluminium, le silicium, le titane et le carbone, à concurrence de 0 à 25 % en poids, toutes les quantités se rapportant au poids total de la poudre métallique.

2. Procédé selon la revendication 1, **caractérisé en ce que** les poudres métalliques contiennent un ou plusieurs métaux du fer à concurrence de 70 à 100 % en poids, de préférence de 80 à 100 % en poids.

3. Procédé selon la revendication 1, **caractérisé en ce que** les poudres métalliques possèdent une teneur de 0,1 à 15 % en poids, de préférence de 0,3 à 10 % en poids, de manière particulièrement préférée de 0,5 à 5 % en poids en un ou plusieurs métaux du VIème sous-groupe.

4. Procédé selon la revendication 1, **caractérisé en ce que** les poudres métalliques possèdent une teneur de 0 à 10 % en poids en aluminium et de 0 à 5 % en poids des éléments respectifs Si, Ti et C.

5. Procédé selon la revendication 4, **caractérisé en ce que** la teneur totale des éléments Al, Si, Ti et C s'élève de 0 à 15 % en poids, de préférence de 0 à 10 % en poids.

6. Procédé selon la revendication 1, **caractérisé en ce que** la pression d'hydrogène s'élève de 5 à 400 bar, de préférence de 100 à 350 bar, de manière particulièrement préférée de 100 à 300 bar.

7. Procédé selon la revendication 1, **caractérisé en ce que** la température d'hydrogénation s'élève de 140 à 280°C, de préférence de 180 à 260°C.

8. Procédé selon la revendication 1, **caractérisé en ce qu**'au cours du procédé, par mole de la matière de départ, on met en oeuvre une quantité d'hydrogène supérieure de 10 à 60 fois, de préférence de 20 à 40 fois, rapportée à la quantité stoechiométrique.

9. Procédé selon la revendication 1, **caractérisé en ce qu**'on travaille en continu en phase liquide descendante ou de préférence en phase liquide montante sur des catalyseurs en lit fixe et on règle une charge de catalyseur de 0,05 à 1,0 kg, de préférence de 0,1 à 0,5 kg du produit de départ, par litre de catalyseur et par heure.

10. Procédé selon la revendication 1, **caractérisé en ce qu**'on dilue le produit de départ avec un solvant inerte vis-à-vis de la réaction à concurrence de 10 à 100 % en poids, de préférence de 10 à 40 % en poids, rapportés au produit de départ.
